# EUROPEAN PATENT APPLICATION

(11) **EP 4 831 185 A1**
(43) Date of publication of application: **28.10.2026**
(21) Application number: 25732862.5
(22) Date of filing: 21.01.2025
(51) Int. Cl.: C07F 9/09, C07D 241/12, C07D 233/64, C07C 229/26, C07C 279/14, C07C 281/16, A61P 9/10, A61P 9/00, A61K 31/661

(54) **NOVEL IDEBENONE PHOSPHATE, SALT COMPOUND THEREOF, PREPARATION METHOD THEREFOR AND USE THEREOF**

(30) Priority: 21.12.2023 CN 202311779617
(71) Applicant: Shanghai Institute of Pharmaceutical Industry Co., Ltd., Shanghai, 200040 (CN); China State Institute of Pharmaceutical Industry Co,. Ltd., China (Shanghai) Pilot Free Trade Zone Pudong New Area, Shanghai 201203 (CN)
(72) Inventor: WANG, Guan, Beijing Road Jingan District, Shanghai 200040 (CN); LIU, Li, Beijing Road Jingan District, Shanghai 200040 (CN); JIA, Jian, Beijing Road Jingan District, Shanghai 200040 (CN); WANG, Huihao, Beijing Road Jingan District, Shanghai 200040 (CN)
(74) Representative: IK-IP LTD
(86) International application number: PCT/CN2025/073543
(87) International publication number: WO 2025/131134

(57) **Abstract**

Disclosed in the present invention are a novel idebenone phosphate, a salt compound thereof, a preparation method therefor and a use thereof. Provided in the present invention is a compound represented by formula (I) or a pharmaceutically acceptable salt thereof. The novel idebenone phosphate and the salt compound thereof can significantly improve the water solubility of an idebenone technical material. By using the form of a phosphate or the salt of a phosphate ester and a basic bioactive molecule, the ability of same to penetrate through a blood-brain barrier is improved, and the solution can collaboratively have the effects of anti-cerebral infarction, anti-cerebral edema, neuroprotection, vascular endothelium protection or improved microcirculation, etc., and can be used in drugs for preventing or treating ischemic cerebrovascular diseases and improving cerebral blood circulation disorder, especially for acute phase administration treatment in ischemic stroke.

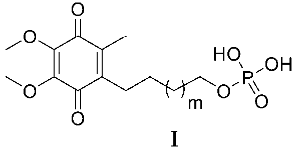

## Description

### SPECIFICATION

This application claims priority to Chinese Patent Application No. 202311779617.3, filed on December 21, 2023. The aforementioned Chinese patent application is incorporated herein by reference in its entirety.

### TECHNICAL FIELD

The present disclosure belongs to the field of medicinal chemistry and pharmacotherapeutics, and specifically relates to novel idebenone phosphate and a salt compound thereof, a preparation method therefor, and a use thereof, particularly a use of idebenone phosphate arginine salt in the manufacture of a medicament for preventing or treating an ischemic cerebrovascular disease or cerebral blood circulation disorder, especially a use in the acute phase treatment thereof.

### BACKGROUND

Stroke represents a class of diseases that seriously endangers human health and life, and has become one of the diseases with the highest mortality and disability rates worldwide. According to the neurovascular unit (NVU) theory, an ideal anti-stroke drug should possess dual effects of neuroprotection and of improving cerebral blood flow microcirculation. Currently marketed drugs usually exert therapeutic efficacy only through mechanisms such as improving cerebral blood flow microcirculation (e.g., butylphthalide), scavenging free radicals (e.g., edaravone), and anti-excitotoxicity, and have limited therapeutic effects; therefore, there is an urgent clinical need for more effective new therapeutic drugs.

Coenzyme Q10 (CoQ10) is an important electron transport carrier and antioxidant in the mitochondrial oxidative respiratory chain, and is used in the treatment of cardiovascular diseases, hepatitis, and the comprehensive treatment of cancer. Idebenone (IDB) is a synthetic analog of coenzyme Q10, and its antioxidant capacity is more than 100 times that of coenzyme Q10. Its chemical name is 6-(10-hydroxydecyl)-2,3-dimethoxy-5-methyl-1,4-benzoquinone, with a molecular formula of C₁₉H₃₀O₅, and a molecular weight of 338.44. The chemical structural formula thereof is shown in formula 1:

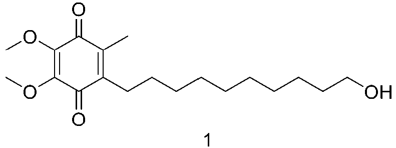

In 2017, idebenone tablets were marketed in China for the treatment of brain function impairment caused by chronic cerebrovascular diseases, traumatic brain injuries, and the like. It can improve mental and behavioral disorders such as subjective symptoms, language, anxiety, depression, memory loss, and decline in intelligence. The product is a drug for improving brain metabolism and mental symptoms, which can activate brain mitochondrial respiratory activity, improve brain energy metabolism in cerebral ischemia, improve glucose utilization rate in the brain, increase ATP production in the brain, inhibit the generation of lipid peroxides by brain mitochondria, and inhibit membrane disorders caused by lipid peroxidation of brain mitochondrial membranes. The product has low toxic and side effects. Specifically, the oral LD₅₀ in both rats and mice is greater than 10000 mg/kg. No obvious toxic reaction has been observed in dogs after oral administration of 100 mg/(kg·d) for 1 year, and it has no teratogenic, carcinogenic, or mutagenic effects. The product is extremely difficult to dissolve in water and is easily soluble in organic solvents. Clinically, it is an oral tablet, with a dosage of 30 mg (1 tablet) per administration for adults, three times a day. However, the bioavailability is low, and only a 1% dose can reach the systemic blood circulation.

Idebenone exerts strong antioxidant and free radical scavenging effects by activating mitochondrial oxidative function, and possesses the additional effect of improving cerebral microcirculation blood flow, which, in theory, can be developed into an ideal new drug for treating stroke. However, due to the extremely poor water solubility of idebenone, it is currently administered exclusively orally. Following oral administration, with a first-pass effect of the liver and intestinal mucosa, only 1% of the dose can reach the systemic blood circulation, resulting in a very low bioavailability. Therefore, although its brain permeability is good, due to the low plasma exposure, it is difficult to achieve a high therapeutic concentration in the brain, which largely limits its application in brain diseases. If the pharmacokinetic properties of idebenone can be further improved to increase its water solubility, increase the plasma exposure of the drug, and maintain its good brain permeability, thereby enhancing its pharmacodynamic activity, it would be of great significance for the development of drugs for preventing or treating ischemic cerebrovascular diseases or cerebral blood circulation disorders.

L-Arginine (L-Arg) is a basic amino acid with a molecular formula of C₆H₁₄N₄O₂. It is a semi-essential amino acid that exists abundantly in protamine and the like, and is a basic constituent of various proteins. As a substrate of nitric oxide synthase *in vivo,* L-arginine can be converted *in vivo* into nitric oxide, which is beneficial to the cardiovascular and cerebrovascular systems. Literature reports that L-arginine can enter the brain from the circulating blood via cationic amino acid transporters on the blood-brain barrier, and is extremely important for brain development (Microvasc Res, 2018, 117: 16-21.). It is also reported that intravenous infusion or oral administration of L-arginine in patients with MELAS-type mitochondrial encephalopathy can also increase cerebral blood flow microcirculation, reduce acute injury from focal cerebral ischemia, and decrease the frequency and severity of stroke symptoms (Neurology, 2005, 64(4): 710-712).

### SUMMARY

The technical problem to be solved by the present disclosure is that idebenone or an analog thereof has poor solubility, low drug plasma exposure, and very low bioavailability, with a view to overcoming the deficiencies in clinical therapeutic effects of existing anti-stroke drugs. For this purpose, the present disclosure provides a novel idebenone phosphate and a salt compound thereof, a preparation method therefor, and a use thereof, aiming to substantially improve the water solubility of the idebenone original drug, adopting the form of a phosphate ester salt or a salt of a phosphate ester with a basic biologically active molecule to improve its ability to penetrate the blood-brain barrier, and synergistically exerting efficacies such as anti-cerebral infarction, anti-cerebral edema, neuroprotection, vascular endothelial protection, or improvement of blood flow microcirculation, for use in a medicament for preventing or treating ischemic cerebrovascular diseases and improving cerebral blood circulation disorders, especially for administration and treatment in the acute phase of ischemic stroke.

The present disclosure provides a compound of formula I or a pharmaceutically acceptable salt thereof, 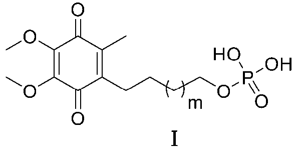 wherein m is 0, 1, 2, 3, 4, 5, 6, 7, or 8.

In one embodiment, the pharmaceutically acceptable salt of the compound of formula I may be a compound of formula II, 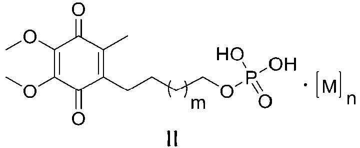
n is 1 or 2;
M is a monovalent metal ion, a divalent metal ion, an organic base, a basic amino acid, or an active alkaloid.

In one embodiment, M may be a monovalent metal ion, an organic base, a basic amino acid, or an active alkaloid.

In one embodiment, the monovalent metal ion may be one or two selected from potassium ion and sodium ion, for example, potassium ion or sodium ion.

In one embodiment, the organic base may be one, two, or three selected from ammonia water, meglumine, and aminoguanidine, for example, ammonia water, meglumine, or aminoguanidine.

In one embodiment, the basic amino acid may be one, two, or three selected from arginine, lysine and histidine, for example, arginine, lysine, or histidine, further for example, L-arginine, L-lysine, or L-histidine.

In one embodiment, the active alkaloid may be tetramethylpyrazine.

In one embodiment, the compound of formula I or a pharmaceutically acceptable salt thereof may be selected from the following compounds:

| Compound No. | Chemical structure |
|---|---|
| I-1 | 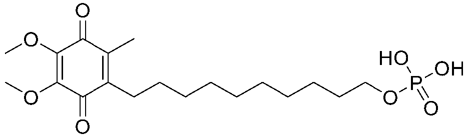 10-(4,5-dimethoxy-2-methyl-3,6-dioxocyclohexa-1,4-dien-1-yl)decyl phosphate |
| I-2 | 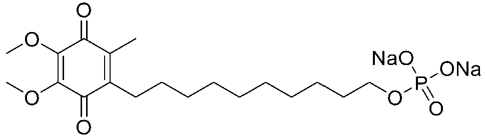 sodium 10-(4,5-dimethoxy-2-methyl-3,6-dioxocyclohexa-1,4-dien-1-yl)decyl phosphate |
| I-3 | 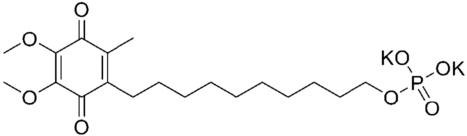 potassium 10-(4,5-dimethoxy-2-methyl-3,6-dioxocyclohexa-1,4-dien-1-yl)decyl phosphate |
| I-4 | 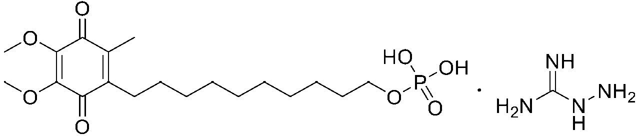 aminoguanidine 10-(4,5-dimethoxy-2-methyl-3,6-dioxocyclohexa-1,4-dien-1-yl)decyl phosphate |
| I-5 | 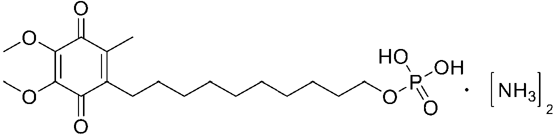 ammonium 10-(4,5-dimethoxy-2-methyl-3,6-dioxocyclohexa-1,4-dien-1-yl)decyl phosphate |
| I-6 | 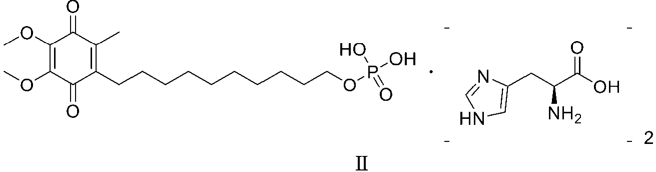 L-histidine 10-(4,5-dimethoxy-2-methyl-3,6-dioxocyclohexa-1,4-dien-1-yl)decyl phosphate |
| I-7 | 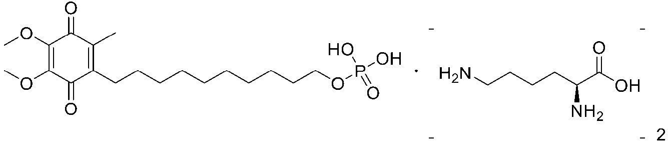 L-lysine 10-(4,5-dimethoxy-2-methyl-3,6-dioxocyclohexa-1,4-dien-1-yl)decyl phosphate |
| I-8 | 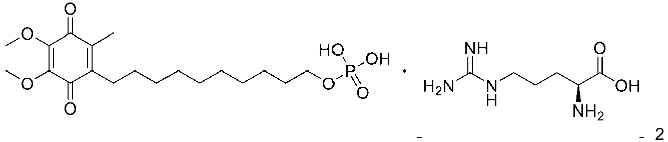 L-arginine 10-(4,5-dimethoxy-2-methyl-3,6-dioxocyclohexa-1,4-dien-1-yl)decyl phosphate |
| I-9 | 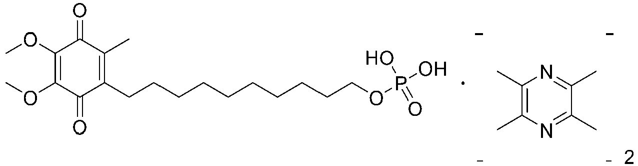 tetramethylpyrazine 10-(4,5-dimethoxy-2-methyl-3,6-dioxocyclohexa-1,4-dien-1-yl)decyl phosphate |

The present disclosure provides a preparation method for the above-mentioned compound of formula I, comprising the following step: subjecting a compound of formula I-1 and pyrophosphoryl chloride to a reaction as shown in the scheme below in a solvent to obtain the compound of formula I, 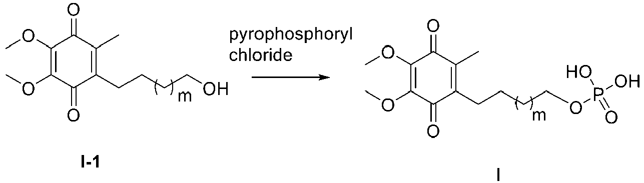 m may also be as defined in any one of the preceding embodiments.

**In** the preparation method for the compound of formula I, the solvent is a conventional solvent for such reactions in the art; preferably, the solvent is an organic solvent, for example, one or more selected from tetrahydrofuran, acetonitrile, dichloromethane, trimethyl phosphate, and triethyl phosphate, and further for example, tetrahydrofuran.

**In** one embodiment, in the preparation method for the compound of formula I, the volume-to-mole ratio of the solvent to the compound of formula I-1 may be 5 to 50 L/mol, for example, 10 L/mol.

In one embodiment, in the preparation method for the compound of formula I, the molar ratio of the pyrophosphoryl chloride to the compound of formula I-1 may be (1 to 2):1, for example, 1.5:1.

In one embodiment, in the preparation method for the compound of formula I, the pyrophosphoryl chloride may be added to a system of the reaction in the form of a tetrahydrofuran solution of pyrophosphoryl chloride; preferably, the volume-to-mole ratio of tetrahydrofuran to pyrophosphoryl chloride in the tetrahydrofuran solution of pyrophosphoryl chloride may be (1 to 2) L/mol, for example, 1.3 L/mol.

In one embodiment, in the preparation method for the compound of formula I, the pyrophosphoryl chloride may be added to a system of said reaction by dropwise addition; the temperature during the dropwise addition may be -20°C to -45°C, for example, -30°C.

In one embodiment, in the preparation method for the compound of formula I, the temperature of the reaction may be -20°C to -45°C, for example, -20°C to 30°C.

In the preparation method for the compound of formula I, the progress of the reaction may be monitored using a conventional detection method for such reactions in the art (e.g., HPLC, TLC, or LC-MS), with the disappearance of the compound of formula I-1 or cessation of the reaction serving as an endpoint of the reaction. The duration of the reaction may be 4 to 8 hours, for example, 6 hours.

In the preparation method for the compound of formula I, after the reaction is completed, the preparation method may further comprise a post-treatment step, wherein the post-treatment step may be a conventional post-treatment step for such reactions in the art, for example, one or more selected from the following steps: concentration, extraction, washing, pH adjustment, and drying; for example, including concentration, extraction, washing, pH adjustment, and drying; a solvent for said extraction may be ethyl acetate; said washing may be performed with water and a saturated aqueous NaHCO₃ solution; and said pH adjustment may involve adjusting the pH to between 1 and 2 using concentrated hydrochloric acid.

The present disclosure provides a preparation method for the above-mentioned compound of formula II, comprising the following step: subjecting the compound of formula I and a basic substance to a reaction as shown in the scheme below in water to obtain the compound of formula II, 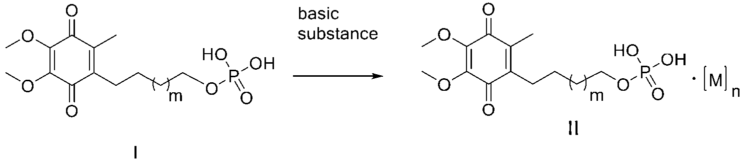 the basic substance is a metal salt, an organic base, a basic amino acid, or an active alkaloid; metal in the metal salt is a monovalent metal ion or a divalent metal ion, and M, m, and n may be as defined in any one of the preceding embodiments.

The monovalent metal ion, the divalent metal ion, the organic base, the basic amino acid, and the active alkaloid may also be as defined in any embodiment of the present disclosure.

In one embodiment, the metal in the metal salt is a monovalent metal ion; the metal salt may be one or more selected from sodium bicarbonate, sodium carbonate, sodium hydroxide, potassium bicarbonate, potassium carbonate, and potassium hydroxide, for example, sodium carbonate or potassium bicarbonate.

In one embodiment, the water may be ionized water or purified water.

In one embodiment, the volume-to-mole ratio of the water to the compound of formula I is (5 to 100) L/mol, for example, (5 to 50) L/mol, further for example, 12 L/mol, 17.5 L/mol, 18 L/mol, 18.5 L/mol, 20 L/mol, 27 L/mol, or 47 L/mol.

In one embodiment, the molar ratio of the basic substance to the compound of formula I may be 1:1 or 2:1.

In one embodiment, in the preparation method for the compound of formula II, the temperature of the reaction may be 10°C to 40°C, for example, 30°C to 35°C or 20°C to 30°C.

In one embodiment, in the preparation method for the compound of formula II, the duration of the reaction may be 20 to 60 minutes, for example, 30 minutes.

In the preparation method for the compound of formula II, after the reaction is completed, the preparation method further comprises a post-treatment step, wherein the post-treatment step is a conventional post-treatment step for such reactions in the art, for example, lyophilization.

The present disclosure provides a pharmaceutical composition comprising:
(1) (a therapeutically effective amount of) the compound of formula I as described in any embodiment of the present disclosure or a pharmaceutically acceptable salt thereof, and
(2) a pharmaceutical excipient.

The present disclosure provides a use of a substance X in the manufacture of a medicament for preventing and/or treating an ischemic cerebrovascular disease or cerebral blood circulation disorder, wherein the substance X is the compound of formula I as described in any embodiment of the present disclosure, a pharmaceutically acceptable salt thereof, or the above-mentioned pharmaceutical composition. The ischemic cerebrovascular disease or cerebral blood circulation disorder may be cerebral infarction, stroke, or cerebral microcirculatory disease.

The present disclosure provides a method for preventing and/or treating an ischemic cerebrovascular disease or cerebral blood circulation disorder, comprising administering to a subject a therapeutically effective amount of a substance X, wherein the substance X is the compound of formula I as described in any embodiment of the present disclosure, a pharmaceutically acceptable salt thereof, or the above-mentioned pharmaceutical composition.

The present disclosure further provides a substance X for use as a medicament, wherein the substance X is the compound of formula I as described in any embodiment of the present disclosure, a pharmaceutically acceptable salt thereof, or the above-mentioned pharmaceutical composition.

The present disclosure further provides a substance X for use in treatment, wherein the substance X is the compound of formula I as described in any embodiment of the present disclosure, a pharmaceutically acceptable salt thereof, or the above-mentioned pharmaceutical composition.

The present disclosure further provides a substance X for use in the prophylaxis and/or treatment of an ischemic cerebrovascular disease or cerebral blood circulation disorder, wherein the substance X is the compound of formula I as described in any embodiment of the present disclosure, a pharmaceutically acceptable salt thereof, or the above-mentioned pharmaceutical composition.

Without departing from common knowledge in the art, the above preferred conditions can be arbitrarily combined to obtain various preferred embodiments of the present disclosure.

Unless otherwise specified, the terms used in the present disclosure have the following meanings:

The term "pharmaceutically acceptable salt" refers to a salt prepared from a compound of the present disclosure with a relatively non-toxic, pharmaceutically acceptable acid or base.

The term "pharmaceutical excipient" refers to the vehicles and adjuvants used in the manufacture of pharmaceutical products and the formulation of prescriptions. They include all substances contained in a pharmaceutical preparation, except for the active ingredients. See the Pharmacopoeia of the People's Republic of China (2015 Edition, Part IV) or the Handbook of Pharmaceutical Excipients (Raymond C Rowe, 2009 Sixth Edition).

The term "treatment" refers to remedial therapy. When relating to a specific condition, treatment refers to: (1) alleviating one or more biological manifestations of a disease or condition, (2) interfering with (a) one or more points in a biological cascade that leads to or causes the condition or (b) one or more biological manifestations of the condition, (3) ameliorating one or more symptoms, effects, or side effects associated with the condition, or one or more symptoms, effects, or side effects associated with the condition or the treatment thereof, or (4) slowing the progression of the condition or of one or more biological manifestations of the condition.

The term "prevention" refers to a reduction in the risk of acquiring or developing a disease or disorder.

The term "therapeutically effective amount" refers to an amount of a compound that, when administered to a patient, is sufficient to effectively treat a disease or condition described herein. The "therapeutically effective amount" will vary depending on the compound, the condition and its severity, and the age of the patient to be treated, but can be adjusted as needed by a person skilled in the art.

The reagents and raw materials used in the present disclosure are all commercially available.

The advantageous effects of the present disclosure lie in that: the present disclosure provides a novel water-soluble idebenone phosphate compound (I) and a salt thereof, which belongs to a class of substances easily soluble in water, thereby being capable of increasing drug plasma exposure, enhancing the pharmacodynamic activity thereof, and being more favorable for a drug for preventing and/or treating ischemic cerebrovascular diseases or cerebral blood circulation disorders.

Compound I-8 of the present disclosure further has the following excellent properties: (1) possessing basic characteristics of a prodrug, being capable of metabolizing *in vivo* and releasing idebenone and L-arginine that exert a synergistic effect, significantly enhancing the therapeutic effect against acute ischemic stroke; (2) the idebenone phosphate amino acid salt has significant transcellular and blood-brain barrier-penetrating effects, increasing the blood drug concentration in the brain; (3) L-arginine metabolizes in the brain to generate a low concentration of nitric oxide, exerting an effect of dilating cerebral blood vessels; (4) potent neurological function protection activity, as well as anti-cerebral infarction and anti-cerebral edema activities; (5) possessing good water solubility, and being capable of being developed into an injection for treatment in the acute phase of ischemic stroke, which widens its clinical applicability on the basis of further improving the drug activity of idebenone.

### BRIEF DESCRIPTION OF THE DRAWINGS

FIG. 1 shows the effects of the tested compounds of each group on OGD/R-induced SH-SY5Y cell injury.
FIG. 2 shows the effects of the tested substances of each group on the cerebral infarct volume of tMCAO rats.
FIG. 3 shows TTC staining images of the brains of tMCAO rats after administration of the tested substances of each group.

### DETAILED DESCRIPTION OF THE PREFERRED EMBODIMENT

The present disclosure will be further described with reference to the following examples, but the present disclosure is not intended to be limited to the scope of these examples. In the following examples, experimental methods for which specific conditions are not indicated shall be carried out in accordance with conventional methods and conditions, or selected based on the product instructions.

### Example 1: Synthesis of 10-(4,5-dimethoxy-2-methyl-3,6-dioxocyclohexa-1,4-dien-1-yl)decyl phosphate

Idebenone (10 mmol, 3.38 g) was dissolved in tetrahydrofuran (80 mL), and the temperature was lowered to -20 to -30°C with stirring for later use. Pyrophosphoryl chloride (15 mmol, 3.78 g) was diluted in tetrahydrofuran (20 mL), and the solution was added dropwise to the above idebenone tetrahydrofuran solution. After the dropwise addition was completed, the reaction was continued at -20 to -30°C for 6 hours, and the reaction progress was monitored by TLC (PE:EA = 1:1). At the end of the reaction, the reaction solution was concentrated under vacuum to remove tetrahydrofuran, followed by adding ethyl acetate (100 mL) for extraction, and washing with water (40 mL) twice. The separated aqueous phase was discarded. A saturated aqueous NaHCO₃ solution (60 mL) was added to the organic phase for washing twice. The aqueous layers were combined, and further extracted with ethyl acetate (40 mL) to obtain an aqueous phase by liquid separation. The pH of the aqueous phase was adjusted to 1 to 2 with concentrated hydrochloric acid, followed by extraction with ethyl acetate (60 mL * 3). The organic layers were combined, dried over anhydrous sodium sulfate, and concentrated to dryness under reduced pressure to obtain 2.85 g of 10-(4,5-dimethoxy-2-methyl-3,6-dioxocyclohexa-1,4-dien-1-yl)decyl phosphate as an orange-red solid, with a yield of 68.2%.
¹H-NMR (400 MHz, CDCl₃): δ (ppm): 3.97 (d, J = 0.7 Hz, 6H), 3.89 (td, J = 7.0, 5.6 Hz, 2H), 2.41 (t, J = 7.5 Hz, 2H), 1.99 (s, 3H), 1.75-1.52 (m, 2H), 1.38-1.18 (m, 14H);
ESI-HRMS (m/z): calcd for C₁₉H₃₁O₈P [M+H]⁺ 419.18293, found 419.18227, error ppm -1.58.

### Example 2: Synthesis of sodium 10-(4,5-dimethoxy-2-methyl-3,6-dioxocyclohexa-1,4-dien-1-yl)decyl phosphate, SIPI11523

10-(4,5-Dimethoxy-2-methyl-3,6-dioxocyclohexa-1,4-dien-1-yl)decyl phosphate (221 mg, 0.53 mmol) was dissolved in 25 mL of pure water and stirred without achieving a clear solution. Accurately weighed Na₂CO₃ (56.2 mg, 0.53 mmol) was added, whereupon the reaction solution immediately became clear. The temperature was controlled at 30 to 35°C, and the mixture was stirred for the reaction for 30 minutes. At the end of the reaction, the reaction solution was transferred to a 50 mL Erlenmeyer flask, and freeze-dried in a freeze dryer at -40°C and 35 Pa for 24 hours to obtain 240 mg of an orange-yellow solid, with a yield of 98.4%.
mp 279.7 to 285.6 °C;
¹H-NMR (400 MHz, D₂O): δ (ppm): 3.94 (d, J = 0.7 Hz, 6H), 3.71 (td, J = 7.0, 5.6 Hz, 2H), 2.44 (t, J = 7.5 Hz, 2H), 1.98 (s, 3H), 1.75-1.52 (m, 2H), 1.30-1.16 (m, 14H);
¹³C-NMR (100 MHz, D₂O): δ (ppm): 160.58, 144.27, 143.62, 139.69, 70.16, 64.67, 61.21, 30.42, 28.93, 28.49, 28.01, 25.80, 25.24, 23.24, 11.40;
ESI-MS [M+H]⁺: m/z 419.2.

### Example 3: Synthesis of potassium 10-(4,5-dimethoxy-2-methyl-3,6-dioxocyclohexa-1,4-dien-1-yl)decyl phosphate, SIPI11524

Referring to the synthesis and post-treatment methods in Example 2, 10-(4,5-dimethoxy-2-methyl-3,6-dioxocyclohexa-1,4-dien-1-yl)decyl phosphate (0.509 mmol, 213 mg) was dissolved in 10 mL of pure water, and potassium bicarbonate (1.08 mmol, 101.9 mg) was added in batches, followed by a reaction with stirring at a room temperature of 20 to 30°C for 30 minutes. After post-treatment, 245 mg of an orange-yellow solid was obtained, with a yield of 97.6%.
mp 280.9-286.8 °C;
¹H-NMR (400 MHz, D₂O): δ (ppm): 3.93 (d, J = 0.7 Hz, 6H), 3.72 (td, J = 7.0, 5.6 Hz, 2H), 2.38 (t, J = 7.4 Hz, 2H), 1.92 (s, 3H), 1.61-1.54 (m, 2H), 1.35-1.16 (m, 14H);
¹³C-NMR (100 MHz, D₂O): δ (ppm): 185.85, 160.23, 144.27, 139.63, 70.15, 64.91, 61.20, 30.36, 28.82, 28.03, 25.81, 25.22, 11.41;
ESI-MS [M+H]⁺: m/z 419.2.

### Example 4: Synthesis of aminoguanidine10-(4,5-dimethoxy-2-methyl-3,6-dioxocyclohexa-1,4-dien-1-yl)decyl phosphate, SIPI11525

Referring to the synthesis and post-treatment methods in Example 2, 10-(4,5-dimethoxy-2-methyl-3,6-dioxocyclohexa-1,4-dien-1-yl)decyl phosphate (0.54 mmol, 226 mg) was dissolved in 10 mL of pure water, and aminoguanidine bicarbonate (0.54 mmol, 73.0 mg) was added in batches, followed by a reaction with stirring at a room temperature of 20 to 30°C for 30 minutes. After post-treatment, 250 mg of an orange-yellow solid was obtained, with a yield of 94.0%.
mp 168.2-174.4 °C;
¹H-NMR (400 MHz, D₂O): δ (ppm): 3.87 (d, J = 0.7 Hz, 6H), 3.70 (td, J = 7.0, 5.6 Hz, 2H), 2.35 (d, J = 8.1 Hz, 2H), 1.87 (s, 3H), 1.60-1.47 (m, 2H), 1.35-1.03 (m, 14H);
¹³C-NMR (100 MHz, D₂O): δ (ppm): 185.77, 144.20, 143.35, 65.28, 61.15, 30.34, 29.53 - 28.61 (m), 28.13, 25.25, 11.41;
ESI-MS [M+H]⁺: m/z 419.2.

### Example 5: Synthesis of ammonium 10-(4,5-dimethoxy-2-methyl-3,6-dioxocyclohexa-1,4-dien-1-yl)decyl phosphate, SIPI11526

Referring to the synthesis and post-treatment methods in Example 2, 10-(4,5-dimethoxy-2-methyl-3,6-dioxocyclohexa-1,4-dien-1-yl)decyl phosphate (0.50 mmol, 210 mg) was dissolved in 10 mL of pure water, and 28% aqueous ammonia (1.00 mmol, 60.7 mg) was added in batches, followed by a reaction with stirring at a room temperature of 20 to 30°C for 30 minutes. After post-treatment, 220 mg of an orange-yellow solid was obtained, with a yield of 96.9%.
mp 154.9-160.6 °C;
¹H-NMR (400 MHz, D₂O): δ (ppm): 3.95 (d, J = 1.1 Hz, 6H), 3.83 (dd, J = 13.2, 6.6 Hz, 2H), 2.38 (t, J = 7.4 Hz, 2H), 1.95 (s, 3H), 1.65-1.52 (m, 2H), 1.48-1.18 (m, 14H);
¹³C-NMR (100 MHz, D₂O): δ (ppm): 185.60, 184.88, 144.24, 144.16, 143.28, 139.21, 70.20, 65.69, 30.21, 29.40, 28.74, 28.25, 25.93, 25.23, 11.44;
ESI-MS [M+H]⁺: m/z 419.2.

### Example 6: Synthesis of L-histidine 10-(4,5-dimethoxy-2-methyl-3,6-dioxocyclohexa-1,4-dien-1-yl)decyl phosphate, SIPI11527

Referring to the synthesis and post-treatment methods in Example 2, 10-(4,5-dimethoxy-2-methyl-3,6-dioxocyclohexa-1,4-dien-1-yl)decyl phosphate (0.57 mmol, 240 mg) was dissolved in 10 mL of pure water, and L-histidine (1.14 mmol, 173 mg) was added in batches, followed by a reaction with stirring at a room temperature of 20 to 30°C for 30 minutes. After post-treatment, 400 mg of an orange-yellow solid was obtained, with a yield of 95.6%.
mp 199.3-204.9 °C;
¹H-NMR (400 MHz, D₂O): δ (ppm): 8.01 (s, 2H), 7.14 (s, 2H), 3.98 (dd, J = 7.5, 5.2 Hz, 2H), 3.93 (d, J = 1.0 Hz, 6H), 3.82 (d, J = 7.2 Hz, 2H), 3.29-3.11 (m, 4H), 2.35 (d, J = 8.0 Hz, 2H), 1.92 (s, 3H), 1.65-1.51 (m, 2H), 1.39-1.18 (m, 14H);
¹³C-NMR (100 MHz, D₂O): δ (ppm): 185.83, 185.18, 173.52, 143.38, 139.38, 135.70, 130.93, 116.98, 70.17, 65.56, 61.16, 54.47, 30.13, 29.39, 28.44, 28.11, 27.49, 25.16, 11.41;
ESI-MS [M+H]⁺: m/z 156.2, [M-H]⁺: m/z 417.2.

### Example 7: Synthesis of L-lysine 10-(4,5-dimethoxy-2-methyl-3,6-dioxocyclohexa-1,4-dien-1-yl)decyl phosphate, SIPI11528

Referring to the synthesis and post-treatment methods in Example 2, 10-(4,5-dimethoxy-2-methyl-3,6-dioxocyclohexa-1,4-dien-1-yl)decyl phosphate (0.55 mmol, 230 mg) was dissolved in 10 mL of pure water, and L-lysine (1.10 mmol, 152 mg) was added in batches, followed by a reaction with stirring at a room temperature of 20 to 30°C for 30 minutes. After post-treatment, 380 mg of an orange-yellow solid was obtained, with a yield of 97.4%.
mp 177.5-182.8 °C;
¹H-NMR (400 MHz, D₂O): δ (ppm): 3.87 (d, J = 5.3 Hz, 6H), 3.74-3.64 (m, 2H), 3.47-3.44 (m, 2H), 2.94 (d, J = 5.6 Hz, 4H), 1.92 (d, J = 4.6 Hz, 2H), 1.83 (s, 3H), 1.69-1.57 (m, 6H), 1.28-1.10 (m, 18H);
¹³C-NMR (100 MHz, D₂O): δ (ppm): 174.59, 70.16, 65.50, 61.19, 54.45, 39.01, 30.21, 29.84, 29.31, 28.10, 26.36, 25.19, 23.21, 21.38, 11.43;
ESI-MS [M+H]⁺: m/z 147.2, [M-H]⁺: m/z 417.2.

### Example 8: Synthesis of L-arginine 10-(4,5-dimethoxy-2-methyl-3,6-dioxocyclohexa-1,4-dien-1-yl)decyl phosphate, SIPI11529

Referring to the synthesis and post-treatment methods in Example 2, 10-(4,5-dimethoxy-2-methyl-3,6-dioxocyclohexa-1,4-dien-1-yl)decyl phosphate (0.83 mmol, 350 mg) was dissolved in 10 mL of pure water, and L-arginine (1.67 mmol, 290 mg) was added in batches. A reaction was then carried out with stirring at a room temperature of 20 to 30°C for 30 minutes. After post-treatment, 630 mg of an orange-yellow solid was obtained, with a yield of 98.1%.
mp 173.7-176.1 °C;
¹H-NMR (400 MHz, D₂O): δ (ppm): 3.86 (s, 6H), 3.68-3.61 (m, 2H), 3.44 (t, J = 6.1 Hz, 2H), 3.13 (t, J = 6.8 Hz, 4H), 2.33 (d, J = 7.7 Hz, 2H), 1.89 (s, 3H), 1.73-1.65 (m, 4H), 1.60-1.54 (m, 6H), 1.22-1.16 (m, 14H);
¹³C-NMR (100 MHz, D₂O): δ (ppm): 176.04, 156.75, 70.12, 64.61, 54.47, 40.54, 30.36, 28.82, 28.76, 28.27, 25.25, 23.93;
ESI-MS [M+H]⁺: m/z 175.2, [M-H]⁺: m/z 417.2.

### Example 9: Synthesis of tetramethylpyrazine 10-(4,5-dimethoxy-2-methyl-3,6-dioxocyclohexa-1,4-dien-1-yl)decyl phosphate, SIPI11530

Referring to the synthesis and post-treatment methods in Example 2, 10-(4,5-dimethoxy-2-methyl-3,6-dioxocyclohexa-1,4-dien-1-yl)decyl phosphate (0.37 mmol, 154 mg) was dissolved in 10 mL of pure water, and tetramethylpyrazine (0.74 mmol, 100 mg) was added in batches, followed by a reaction with stirring at a room temperature of 20 to 30°C for 30 minutes. After post-treatment, 235 mg of an orange-yellow solid was obtained, with a yield of 92.3%.
mp 145.6 to 150.8 °C;
¹H-NMR (400 MHz, D₂O): δ (ppm): 3.93 (s, 6H), 3.58-3.38 (m, 2H), 2.38 (t, J = 7.4 Hz, 2H), 2.02 (s, 3H), 1.92 (s, 3H), 1.82-1.74 (m, 9H), 1.61-1.54 (m, 4H), 1.45-1.18 (m, 10H);
¹³C-NMR (100 MHz, D₂O): δ (ppm): 184.83, 184.21, 144.19, 144.10, 142.82, 70.27, 66.22, 60.93, 30.27, 29.84, 28.88, 28.39, 26.01, 25.38, 11.39;
ESI-MS [M+H]⁺: m/z 137.2, [M-H]⁺: m/z 417.2.

### Example 10: Water solubility test of compound I

Referring to the method of the Chinese Pharmacopoeia (2020 Edition), a test substance or idebenone sample ground into a fine powder was weighed and placed in a certain volume of pure water at 25°C±2°C, and vigorously shaken for 30 seconds every 5 minutes; the result of dissolution within 30 minutes was observed, and if no solute particles or droplets were visually observable, it was deemed as completely dissolved.

| | |
|---|---|
| Very soluble | Means that 1 g (ml) of solute can be dissolved in less than 1 ml of solvent; |
| Freely soluble | Means that 1 g (ml) of solute can be dissolved in 1 to less than 10 ml of solvent; |
| Soluble | Means that 1 g (ml) of solute can be dissolved in 10 to less than 30 ml of solvent; |
| Sparingly soluble | Means that 1 g (ml) of solute can be dissolved in 30 to less than 100 ml of solvent; |
| Slightly soluble | Means that 1 g (ml) of solute can be dissolved in 100 to less than 1000 ml of solvent; |
| Very slightly soluble | Means that 1 g (ml) of solute can be dissolved in 1000 to less than 10000 ml of solvent; |
| Practically insoluble or insoluble | Means that 1 g (ml) of solute cannot be completely dissolved in 10000 ml of solvent. |

### Experimental results:

The water solubility test results of compound I are shown in Table 1.

**Table 1. Water solubility test results of the tested substances in each group**

| Compound name | Solubility range | Result | Compound name | Solubility range | Result |
|---|---|---|---|---|---|
| I-1 | 1g/1 to 10ml | Freely soluble | I-6 | 1g/1 to 10ml | Freely soluble |
| I-2 | 1g/1 to 10ml | Freely soluble | I-7 | 1g/1 to 10ml | Freely soluble |
| I-3 | 1g/1 to 10ml | Freely soluble | I-8 | 1g/1 to 10ml | Freely soluble |
| I-4 | 1g/1 to 10ml | Freely soluble | I-9 | 1g/1 to 10ml | Freely soluble |
| I-5 | 1g/1 to 10ml | Freely soluble | Idebenone | <1g/10000ml | Practically insoluble |

The water solubilities of compound I are all freely soluble, whereas idebenone is practically insoluble in water. Compared with idebenone, the water solubility of compound I is significantly improved.

### Example 11: Effects of compound I on in vitro oxygen-glucose deprivation/reoxygenation (OGD/R) model-induced neuroblastoma (SH-SY5Y) cell injury:

### Experimental method:

1) The SH-SY5Y cell line (commercially purchased from KeyGEN BioTECH, Cat. No. KG217) was digested and counted to prepare a cell suspension of 8×10⁴ cells/mL, and 100 µl of the cell suspension was added to each well in a 96-well cell culture plate.
2) The 96-well cell culture plate was placed in an incubator at 37°C with 5% CO₂ and cultured for 24 hours.
3) The drug was diluted with a culture medium to a desired working solution concentration, and 100 µl of the corresponding drug-containing culture medium was added to each well, while a negative control group was established simultaneously, in which the culture medium was replaced with a culture medium containing 1% FBS.
4) The 96-well cell culture plate was placed in an incubator at 37°C with 5% CO₂ and cultured for 24 hours.
5) The culture medium was replaced with a glucose-free and serum-free culture medium, and placed in a 5% CO₂, 95% N₂ environment for hypoxic culture for 2 hours.
6) The culture medium was replaced with a drug-free complete culture medium, and placed in an incubator at 37°C with 5% CO₂ for reoxygenation for 24 hours.
7) The 96-well plate was subjected to CCK-8 staining, and OD values were measured at λ = 450 nm. 10 µl of CCK-8 was added to each well, and culture was continued in the incubator for 2 to 3 hours. The mixture was gently mixed on a shaker for 10 minutes to remove bubbles in the 96-well plate; the OD value of each well was read by a microplate reader at λ = 450 nm, and the inhibition rate was calculated.

### Experimental grouping:

Control group, Model group, edaravone group, idebenone group, and group of idebenone salt compounds to be screened (a total of 8 groups from SIPI11523 to SIPI11530, with n = 3 replicate wells for each group). Concentration selection: 0.3 µM, 1 µM, 3 µM.

### Experimental results:

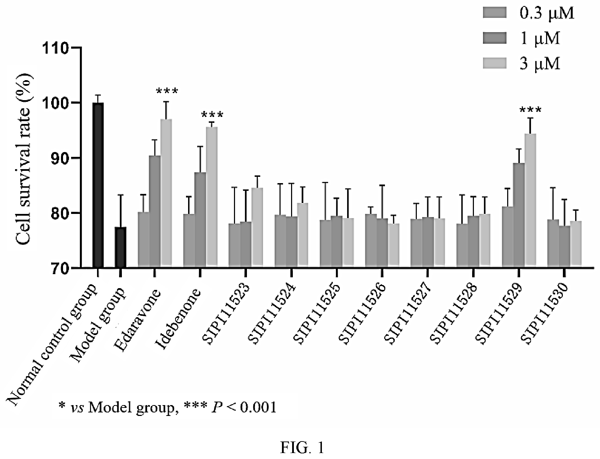

The effects of compound I on OGD/R-induced SH-SY5Y cell injury are shown in FIG. 1 and Table 2. FIG. 1 shows the effects of the tested substances of each group on the cell survival rate. Table 2 shows the effects of the tested substances of each group on the survival rate following cell injury. All data are presented as mean ± SD (n = 3). *** P < 0.001 vs. Model group.

**Table 2. Cell survival rates of OGD/R-induced SH-SY5Y cell injury treated with the tested substances of each group**

| Sample | Cell survival rate (%) | | |
|---|---|---|---|
| | 0.3 µM | 1 µM | 3 µM |
| Cell | 100.00±1.34 | | |
| Cell+OGD/R | 77.42±5.84 | | |
| Edaravone group | 80.19±3.18 | 90.44±2.88 | 97.09±3.14*** |
| Idebenone group | 79.92±3.12 | 87.40±4.72 | 95.71±0.87*** |
| SIPI11523 | 78.12±6.55 | 78.39±5.78 | 84.63±2.14 |
| SIPI11524 | 79.78±5.60 | 79.36±5.99 | 81.86±2.93 |
| SIPI11525 | 78.81±6.75 | 79.50±3.21 | 79.09±5.26 |
| SIPI11526 | 79.92±1.24 | 78.95±6.06 | 78.12±1.49 |
| SIPI11527 | 78.95±2.82 | 79.22±3.67 | 79.09±3.91 |
| SIPI11528 | 78.12±5.20 | 79.50±3.46 | 79.78±3.12 |
| SIPI11529 | 81.30±3.20 | 89.20±2.56 | 94.46±2.81*** |
| SIPI11530 | 78.81±5.78 | 77.70±4.74 | 78.67±1.97 |

As can be seen from FIG. 1 and Table 2, in the *in vitro* constructed oxygen/glucose deprivation-reoxygenation model of human neuroblastoma, SIPI11529 can dose-dependently inhibit OGD/R-induced SH-SY5Y cell injury. The cell survival rate at a concentration of 3 µM is 94.46%, which is superior to that of the other idebenone salt compounds. The protective effect of SIPI11529 (IDBARG) on neuronal cells is comparable to that of idebenone and edaravone, and exhibits good dose dependence.

Conclusion: SIPI11529 can dose-dependently inhibit OGD/R-induced SH-SY5Y cell injury, with an effect comparable to that of the positive control drugs edaravone and idebenone. This suggests that idebenone phosphate arginine salt may cross the cell membrane via active transport, and exert an ability to resist OGD/R-induced SH-SY5Y cell injury.

### Example 12: Test of the effects of SIPI11529 on cerebral infarct area in rats with focal cerebral ischemia

SPF-grade Wistar rats, sourced from Shanghai SLAC Laboratory Animal Co., Ltd., male, with a mean body weight of 220 to 240 g, were housed in an SPF-grade breeding environment, with the indoor temperature maintained in the range of 21 to 25°C and the humidity maintained in the range of 40% to 70%. Artificial lighting was adopted in the animal room, with a 12-hour light/dark cycle (except when working lighting needed to be turned on due to experimental operations, cleaning, etc.), and the experimental animals had free access to food and water.

### Experimental grouping:

Model group: The middle cerebral artery of rats was occluded by a transient middle cerebral artery occlusion (tMCAO) reperfusion suture method. After 90 min of ischemia, the suture was removed to achieve reperfusion. Immediately after reperfusion, physiological saline was injected via the tail vein at an administration volume of 2 mL/kg (n = 10).

Idebenone group: 9.5 mg/kg. 30 Minutes before tMCAO modeling, the tested substance idebenone was administered intragastrically at an administration volume of 10 mL/kg. The middle cerebral artery of the rats was occluded by the tMCAO reperfusion suture method. After 90 minutes of ischemia, the suture was removed to achieve reperfusion (n = 10).

Butylphthalide group: 5.3 mg/kg. The middle cerebral artery of rats was occluded by the tMCAO reperfusion suture method. After 90 minutes of ischemia, the suture was removed to achieve reperfusion. Immediately after reperfusion, butylphthalide injection was injected via the tail vein at an administration volume of 2 mL/kg (n = 10).

L-Arginine group: 9.8 mg/kg. The middle cerebral artery of rats was occluded by the tMCAO reperfusion suture method. After 90 min of ischemia, the suture was removed to achieve reperfusion. Immediately after reperfusion, an L-arginine solution was injected via the tail vein at an administration volume of 2 mL/kg (n = 10).

Idebenone + L-arginine: Idebenone 9.5 mg/kg, L-arginine 9.8 mg/kg. 30 minutes before tMCAO modeling, the tested substance idebenone was administered intragastrically at an administration volume of 10 mL/kg. The middle cerebral artery of rats was occluded by the tMCAO reperfusion suture method. After 90 min of ischemia, the suture was removed to achieve reperfusion. Immediately after reperfusion, an L-arginine solution was injected via the tail vein at an administration volume of 2 mL/kg (n = 10).

SIPI11523 (IDBNA) group: 13 mg/kg. The middle cerebral artery of rats was occluded by the tMCAO reperfusion suture method. After 90 min of ischemia, the suture was removed to achieve reperfusion. Immediately after reperfusion, an IDBNA solution was injected via the tail vein at an administration volume of 2 mL/kg (n = 10).

SIPI11529 (IDBARG) low-dose group: 10 mg/kg. The middle cerebral artery of rats was occluded by the tMCAO reperfusion suture method. After 90 min of ischemia, the suture was removed to achieve reperfusion. Immediately after reperfusion, an idebenone phosphate arginine salt physiological saline solution was injected via the tail vein at an administration volume of 2 mL/kg (n = 10).

SIPI11529 (IDBARG) medium-dose group: 20 mg/kg. The middle cerebral artery of rats was occluded by the tMCAO reperfusion suture method. After 90 min of ischemia, the suture was removed to achieve reperfusion. Immediately after reperfusion, an idebenone phosphate arginine salt physiological saline solution was injected via the tail vein at an administration volume of 2 mL/kg (n = 10).

SIPI11529 (IDBARG) high-dose group: 40 mg/kg. The middle cerebral artery of rats was occluded by the tMCAO reperfusion suture method. After 90 min of ischemia, the suture was removed to achieve reperfusion. Immediately after reperfusion, an idebenone phosphate arginine salt physiological saline solution was injected via the tail vein at an administration volume of 2 mL/kg (n = 10).

The middle cerebral artery of the rats was first occluded by a suture method, and reperfusion was performed after 90 minutes. Immediately after reperfusion, the drug solutions prepared above and an equal volume of physiological saline solution were respectively injected into the rats via the tail vein. The duration of administration for all tested substances was controlled at approximately 1 minute. At 24 hours after ischemia, the rats in each group were sacrificed, and the whole brains were removed for TTC (2,3,5-triphenyltetrazolium chloride) staining to determine the effects of the tested substances of each group on cerebral infarction.

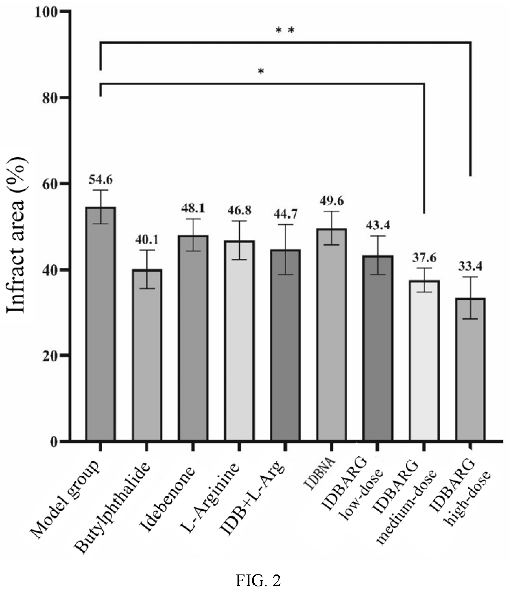

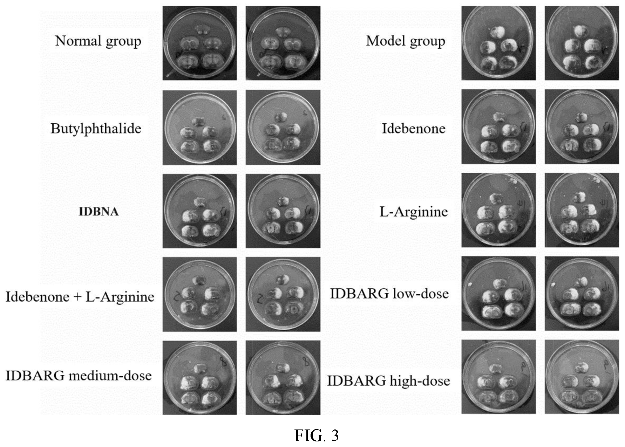

Experimental results: As shown in FIG. 2: Effects of the tested substances of each group on the cerebral infarct area in tMCAO rats. FIG. 3 is a TTC staining image of the brains of tMCAO rats after administration of the tested substances of each group. All data are expressed as mean ± SD (n = 10). * *P* < 0.05 *vs* Model group, ***P* < 0.01 *vs* Model group.

As can be seen from FIG. 2 and FIG. 3, medium and high doses of IDBARG can significantly reduce the cerebral infarct volume in tMCAO rats (P < 0.05, vs. Model group); as the dose increases, the protective effect on the infarct area of ischemic stroke becomes stronger, exhibiting good dose dependence. The effect is superior to that of compound SIPI11523 (IDBNA) and L-arginine, suggesting that the L-arginine salt can synergize with idebenone to exert a synergistic anti-injury effect in tMCAO rats, which is superior to the SIPI11523 (IDBNA) group, and superior to the clinically commonly used drugs butylphthalide group and idebenone group. The effect is superior to the combined administration of idebenone + L-arginine, suggesting that this compound is able to better penetrate the blood-brain barrier to exert a protective effect.

Observation of toxic side effects: Under the administered doses of each group, no toxic side effects were observed in the experimental animals.

Conclusion: SIPI11529 (IDBARG) can ameliorate injury in tMCAO rats, which is reflected by significantly reducing the cerebral infarct volume of the rats, wherein the SIPI11529 (IDBARG) medium-dose group significantly reduces the cerebral infarct volume of the rats (a reduction of 31.1%, *P*<0.05), and the SIPI11529 (IDBARG) high-dose group significantly reduces the cerebral infarct volume of the rats (a reduction of 38.8%, P<0.01), while exhibiting good dose dependence. The therapeutic effect is significantly superior to that of the clinically commonly used positive drugs butylphthalide and idebenone. The experimental results suggest that compound SIPI11529 has more potent anti-stroke activity.

## Claims

1. A compound of formula I or a pharmaceutically acceptable salt thereof, 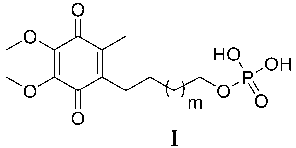 wherein m is 0, 1, 2, 3, 4, 5, 6, 7, or 8.

2. The compound of formula I according to claim 1 or a pharmaceutically acceptable salt thereof, wherein the compound of formula I or the pharmaceutically acceptable salt thereof is a compound of formula II, 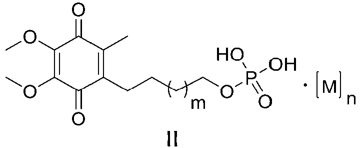
n is 1 or 2;
M is a monovalent metal ion, a divalent metal ion, an organic base, a basic amino acid, or an active alkaloid.

3. The compound of formula I according to claim 2 or a pharmaceutically acceptable salt thereof, wherein the compound of formula II satisfies one or more of the following conditions:
(1) the monovalent metal ion is one or two selected from a potassium ion and a sodium ion, for example, a potassium ion or a sodium ion;
(2) the organic base is one, two, or three selected from ammonia water, meglumine, and aminoguanidine, for example, ammonia water, meglumine, or aminoguanidine;
(3) the basic amino acid is one, two, or three selected from arginine, lysine, and histidine, for example, arginine, lysine, or histidine, further for example, L-arginine, L-lysine, or L-histidine; and
(4) the active alkaloid is tetramethylpyrazine.

4. The compound of formula I according to claim 1 or a pharmaceutically acceptable salt thereof, wherein the compound of formula I or the pharmaceutically acceptable salt thereof is any one of the following compounds:
| Compound No. | Chemical structure |
|---|---|
| I-1 | 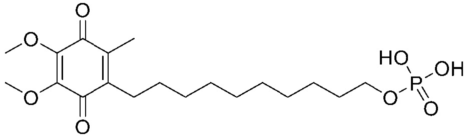 10-(4,5-dimethoxy-2-methyl-3,6-dioxocyclohexa-1,4-dien-1-yl)decyl phosphate |
| I-2 | 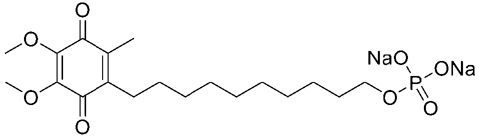 sodium 10-(4,5-dimethoxy-2-methyl-3,6-dioxocyclohexa-1,4-dien-1-yl)decyl phosphate |
| I-3 | 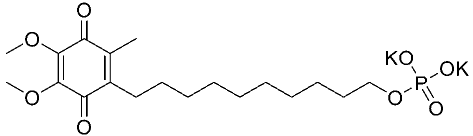 potassium 10-(4,5-dimethoxy-2-methyl-3,6-dioxocyclohexa-1,4-dien-1-yl)decyl phosphate |
| I-4 | 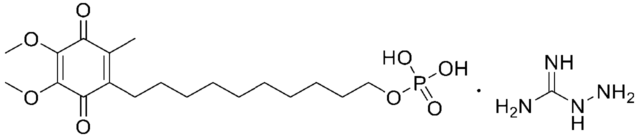 aminoguanidine 10-(4,5-dimethoxy-2-methyl-3,6-dioxocyclohexa-1,4-dien-1-yl)decyl phosphate |
| I-5 | 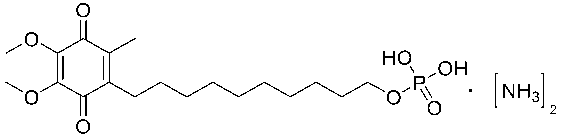 ammonium 10-(4,5-dimethoxy-2-methyl-3,6-dioxocyclohexa-1,4-dien-1-yl)decyl phosphate |
| I-6 | 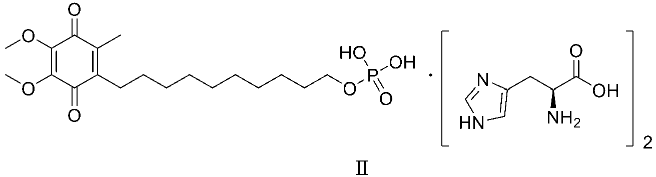 L-histidine 10-(4,5-dimethoxy-2-methyl-3,6-dioxocyclohexa-1,4-dien-1-yl)decyl phosphate |
| I-7 | 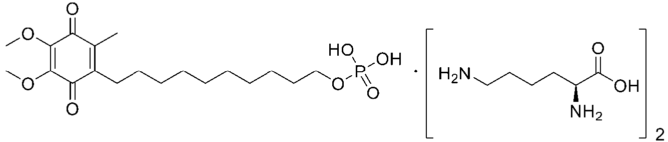 L-lysine 10-(4,5-dimethoxy-2-methyl-3,6-dioxocyclohexa-1,4-dien-1-yl)decyl phosphate |
| I-8 | 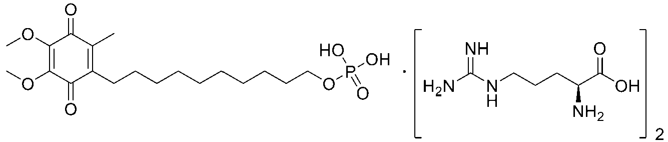 L-arginine 10-(4,5-dimethoxy-2-methyl-3,6-dioxocyclohexa-1,4-dien-1-yl)decyl phosphate |
| I-9 | 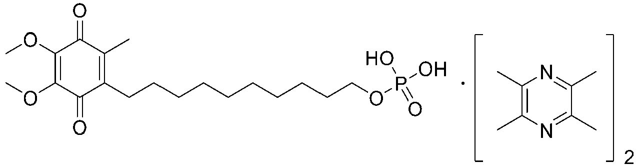 tetramethylpyrazine 10-(4,5-dimethoxy-2-methyl-3,6-dioxocyclohexa-1,4-dien-1-yl)decyl phosphate |

5. A preparation method for a compound of formula I, comprising the following step: subjecting a compound of formula I-1 and pyrophosphoryl chloride to a reaction as shown in the scheme below in a solvent to obtain the compound of formula I, 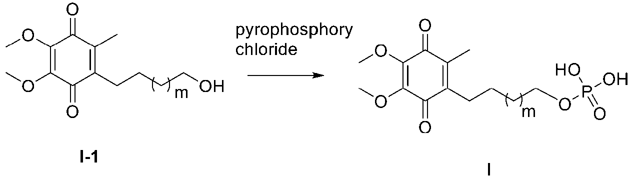 m is as defined in claim 1 or 4.

6. The preparation method for the compound of formula I according to claim 5, wherein the preparation method satisfies one or more of the following conditions:
(1) the solvent is an organic solvent; for example, the solvent is one or more selected from tetrahydrofuran, acetonitrile, dichloromethane, trimethyl phosphate, and triethyl phosphate; further for example, the solvent is tetrahydrofuran;
(2) the volume-to-mole ratio of the solvent to the compound of formula I-1 is (5 to 50) L/mol, for example, 10 L/mol;
(3) the molar ratio of pyrophosphoryl chloride to the compound of formula I-1 is (1 to 2):1, for example, 1.5:1;
(4) the pyrophosphoryl chloride is added to a system of the reaction in the form of a tetrahydrofuran solution of pyrophosphoryl chloride; preferably, the volume-to-mole ratio of tetrahydrofuran to pyrophosphoryl chloride in the tetrahydrofuran solution of pyrophosphoryl chloride is (1 to 2) L/mol, for example, 1.3 L/mol;
(5) the pyrophosphoryl chloride is added to a system of the reaction by dropwise addition; the temperature during the dropwise addition may be -20°C to -45°C, for example, -30°C;
(6) the temperature of the reaction is -20°C to -45°C, for example, -20°C to 30°C; and
(7) after the reaction is completed, the preparation method further comprises a post-treatment step, wherein the post-treatment step is one or more selected from the following: concentration, extraction, washing, pH adjustment, and drying; for example, including concentration, extraction, washing, pH adjustment, and drying; the solvent for the extraction may be ethyl acetate; the washing may be performed with water and a saturated aqueous NaHCO₃ solution; and the pH adjustment may involve adjusting the pH to between 1 and 2 using concentrated hydrochloric acid.

7. A preparation method for a compound of formula II, comprising the following step: subjecting a compound of formula I and a basic substance to a reaction as shown in the scheme below in water to obtain the compound of formula II, 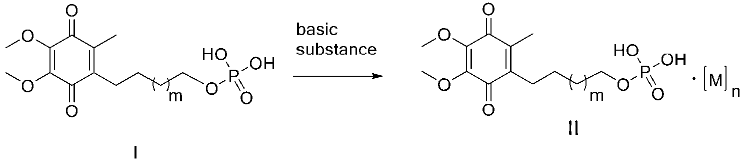
the basic substance is a metal salt, an organic base, a basic amino acid, or an active alkaloid; the metal in the metal salt is a monovalent metal ion or a divalent metal ion; the monovalent metal ion, the organic base, the basic amino acid, and the active alkaloid may also be as defined in claim 3;
m is as defined in claim 1 or 4;
n is as defined in claim 2 or 4;
M is as defined in any one of claims 2 to 4.

8. The preparation method for the compound of formula II according to claim 7, wherein the preparation method satisfies one or more of the following conditions:
(1) the metal in the metal salt is a monovalent metal ion; the metal salt may be one or more selected from sodium bicarbonate, sodium carbonate, sodium hydroxide, potassium bicarbonate, potassium carbonate, and potassium hydroxide, for example, sodium carbonate or potassium bicarbonate;
(2) the water is ionized water or purified water;
(3) the volume-to-mole ratio of the water to the compound of formula I is (5 to 100) L/mol, for example, (5 to 50) L/mol, further for example, 12 L/mol, 17.5 L/mol, 18 L/mol, 18.5 L/mol, 20 L/mol, 27 L/mol, or 47 L/mol;
(4) the molar ratio of the basic substance to the compound of formula I is 1:1 or 2:1;
(5) the temperature of the reaction is 10°C to 40°C, for example, 30°C to 35°C or 20°C to 30°C; and
(6) after the reaction is completed, the reaction further comprises a post-treatment step, for example, lyophilization.

9. A pharmaceutical composition comprising:
(1) the compound of formula I according to any one of claims 1 to 4 or a pharmaceutically acceptable salt thereof, and
(2) a pharmaceutical excipient.

10. Use of a substance X in the manufacture of a medicament for preventing and/or treating an ischemic cerebrovascular disease or cerebral blood circulation disorder; the substance X is the compound of formula I according to any one of claims 1 to 4 or a pharmaceutically acceptable salt thereof, or the pharmaceutical composition according to claim 9; and the ischemic cerebrovascular disease or cerebral blood circulation disorder may be cerebral infarction, stroke, or a cerebral microcirculatory disease.
